# EUROPEAN PATENT SPECIFICATION

(11) **EP 3 180 465 B1**
(45) Date of publication and mention of the grant of the patent: **08.04.2020**
(21) Application number: 15847665.5
(22) Date of filing: 16.09.2015
(51) Int. Cl.: C40B 40/14, C40B 50/14, C40B 60/12

(54) **LIBRARIES OF NON-NATURAL, DIHYDROISOQUINOLINONE-BASED POLYMERS FOR HIGH-THROUGHPUT DRUG SCREENING THROUGH FIBER-OPTIC ARRAY SCANNING TECHNOLOGY**
BIBLIOTHEKEN VON NICHT-NATÜRLICHEN, DIHYDROISOQUINOLINON-BASIERTEN POLYMEREN ZUR WIRKSTOFFENTDECKUNG MIT HOHEM DURCHSATZ MITTELS GLASFASER-ARRAY SCAN-TECHNOLOGIE
BANQUES DE POLYMÈRES NON-NATURELS À BASE DE DIHYDROISOQUINOLINONE POUR LE CIBLAGE DE MÉDICAMENTS À HAUT DÉBIT PAR TECHNOLGIE DE BALAYAGE DE RÉSEAU À FIBRES OPTIQUES

(30) Priority: 16.09.2014 US 201462050922 P
(43) Date of publication of application: 21.06.2017
(73) Proprietor: SRI International, Menlo Park, CA 94025 (US)
(72) Inventor: COLLINS, Nathan, Menlo Park, California 94025 (US); MADRID, Peter, Menlo Park, California 94025 (US)
(74) Representative: Zwicker, Jörk
(86) International application number: PCT/US2015/050306
(87) International publication number: WO 2016/053621

(56) References cited:
- WO-A1-00/12575
- WO-A1-00/45334
- WO-A1-01/14879
- WO-A1-98/39348
- WO-A2-2007/011967
- WO-A2-2009/049098
- US-A- 5 887 278
- US-A1- 2012 077 688
- US-A1- 2013 004 967
- US-A1- 2014 221 241
- US-B2- 7 280 261
- KRITZER J A ET AL: "A Rapid Library Screen for Tailoring . beta .- Peptide Structure and Function", JOURNAL OF THE AMERICAN CHEMICAL SOCIETY, AMERICAN CHEMICAL SOCIETY, US, vol. 127, no. 42, 4 October 2005 (2005-10-04), pages 14584-14585, XP002397201, ISSN: 0002-7863, DOI: 10.1021/JA055050O
- AMIT THAKKAR ET AL: "High-Throughput Sequencing of Peptoids and Peptide-Peptoid Hybrids by Partial Edman Degradation and Mass Spectrometry", JOURNAL OF COMBINATORIAL CHEMISTRY., vol. 11, no. 2, 9 March 2009 (2009-03-09), pages 294-302, XP055389801, US ISSN: 1520-4766, DOI: 10.1021/cc8001734
- MAILLARD, N ET AL.: 'Combinatorial libraries of peptide dendrimers: design, synthesis, on-bead high-throughput screening, bead decoding and characterization.' NAT PROTOC. vol. 4, no. 2, 15 January 2009, pages 132 - 142, XP055357861
- YUE, K ET AL.: 'Constraint-based assembly of tertiary protein structures from secondary structure elements.' vol. 9, no. 10, October 2000, pages 1935 - 1946, XP055357862
- XU, Q ET AL.: 'Protein and chemical microarrays-powerful tools for proteomics.' J BIOMED BIOTECHNOL. vol. 2003, no. 5, 2003, pages 257 - 266, XP008129093
- BUCHSER, W ET AL.: 'Assay development guidelines for image-based high content screening, high content analysis and high content imaging.' ASSAY GUIDANCE MANUAL . 2004, pages 1 - 68, XP055357863

## Description

This invention was made with government support under contract number N66001-14-C-4059 awarded by the Space and Naval Warfare Systems Command Systems Center Pacific. The government has certain rights in this invention

### Introduction

Rapid and cost effective ways of screening large compound collections for biological, physical or chemical properties still remain a challenge in many industries. Pharmaceutical companies have developed high through screening operations which can screen up to 2 million compounds in a matter of months, but these operations require high end automation, compound storage and retrieval system and several FTEs to run and maintain.

Based on a fiber -optic array scanning technology (FAST) developed by SRI for screening for circulating tumor cells (CTCs), we conceived of using the same platform to screen compounds either covalently attached or absorbed onto beads which are similar in size to white blood cells (10 - 20 microns but could by <1 micron or greater than 500 microns) and arraying them on the same glass slide and testing with fluoresence based assays used in the FAST CTC scanner. With this platform we can screen 25,000,000 compounds in -60 secs. A library of 100,000,000 compounds can be screened on four slides in 4 mins.

The cost of reagents (compounds, screening reagents, plates etc.) are greatly reduced using this screening platform. In addition such assays can be run by one technician level person. The invention is of enormous benefit to pharmaceutical companies providing access to cheaper screening and much greater diversity of compound libraries in lead finding though application to rapid discovery of novel affinity reagents for diagnostics and therapeutics as well as catalysts.

Relevant Literature: A rare-cell detector for cancer Krivacic et al. 2004, PNAS 101 (29) 10501-10504; US7280261; US7842465; Multiple biomarker expression on circulating tumor cells in comparison to tumor tissues from primary and metastatic sites in patients with locally advanced/inflammatory, and stage IV breast cancer, using a novel detection technology; Somlo et al., Breast Cancer Res Treat. 2011 Jul;128(1):155-63; Ex Vivo Culture of CTCs: An Emerging Resource to Guide Cancer Maheswaran et al. Therapy Cancer Res June 15, 2015, 75:2411-2415. Kritzer et al (J Am Chem Soc, 2005, 127(42), 14584-14585) relates to the synthesis of libraries of decapeptides coupled to Tentagel beads, i.e. one-bead-one-β-peptide libraries, for rapid library screens. Thakkar et al (J Comb Chem, 2009, 11(2), 294-302) relates to [oligo(N-substitued glycines)] couples to Tentagel beads for high-through put screening. Maillard et al. (Nat Protoc, 2009, 4(2), 132-162) discloses combinatorial libraries of peptide dendrimers produced by solid-phase peptide synthesis. US 2012/0077688 relates to methods for proteomic screening on random protein-bead arrays by mass spec. Photocleavable mass tags are utilized to code a protein library displayed on beads randomly arrayed in an array substrate. A library of probes can be mixed with the protein-bead array to query the array. US 7,280,261 discloses an optical scanner for imaging surfaces having a planar surface for supporting a sample and a bifurcated light path having two fibre optic bundles arranged to define an input and an output of light and a method of using it. WO 01/14879 relates to 4-unsubstituted dihydroisoquinolinone derivatives and combinatorial libraries thereof. US 5,877,278 relates to a two-step solid phase method for the synthesis of N-substituted oligomers such as poly(N-substituted glycines) by (a) assembling glycine monomers and (b) introducing dihyro- or tetrahydroisoquinolines into the sidechains of the glycine oligomers. US 5,877,278 does not disclose that isoquinolines may be used as actual oligomer backbones.

### Summary of the Invention

The invention provides large non-natural polymers of bioactive monomers, and beads, and libraries of beads comprising the polymers. In another aspect, the invention provides use of fiber-optic array scanning technology (FAST) to screen fluorescent beads, including beads comprising the subject polymers. These inventions provide powerful platforms for affinity reagent and catalyst discovery and library on a bead high throughput screening (HTS).

In an aspect, the invention provides a library of beads configured for high-throughput drug screening, each bead comprising affixed non-natural polymers of a distinct bioactive monomer with sequence pre-defined branching and folding in tertiary structures, wherein the monomer is a dihydroisoquinolinone. Any suitable polymer and polymerization may be used that incorporates the requisite bioactive monomer and sequence pre-defined branching and folding in tertiary structures.

In embodiments, the library further comprises, incorporated in each of the polymers secondary structure constraint (SSC) monomers which impose turns and thereby induce the tertiary structures, preferably wherein the SSC monomers are acid cleavable to facilitate mass-spectroscopy based sequencing of the polymers;
the polymer is a polyamide, a vinylogous polymer, vinylogous polyamide, or an ester;
the polymer is polymerized in a coupling reaction selected from Wurtz reaction, Glaser coupling, Ullmann reaction, Gomberg-Bachmann reaction, Cadiot-Chodkiewicz coupling, Pinacol coupling reaction, Castro-Stephens coupling, Gilman reagent coupling, Cassar reaction, Kumada coupling, Heck reaction, Sonogashira coupling, Negishi coupling, Stille cross coupling, Suzuki reaction, Hiyama coupling, Buchwald-Hartwig reaction, Fukuyama coupling, and Liebeskind-Srogl coupling;
the polymer or polymerizations is selected from sulphonamides, reductive aminations, peptoid linkages, Suzuki couplings, phospoamidite couplings, and radical cross coupling;
the monomer is a dihydroisoquinolinone selected from 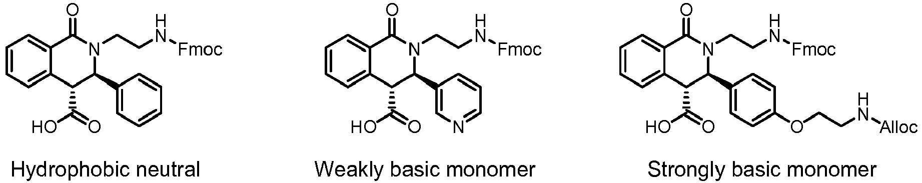 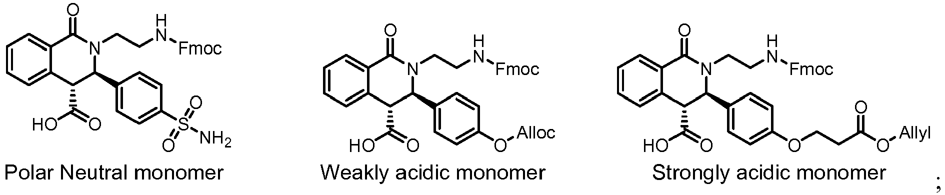 the polymerization combines amide bond coupling methodologies with peptidotriazole branch points introduced using chemically orthogonal copper-catalyzed cycloadditions (click-reactions), and/or
the polymers comprise intrinsically self-readable molecules via intrinsically incorporated isotopically-coded mass-spectroscopy tags or barcodes.

In another aspect, the invention describes an array, which may be ordered in 1, 2 and/or 3-dimensions of a subject library.

In another aspect the invention describes a fiber optic scanner mounted with a slide comprising an ordered array of a subject library.

In another aspect, the invention describes a method of making a subject library comprising affixing the polymers to the beads or building the polymers on the beads by sequential monomer coupling.

In another aspect the invention describes a method of using a fiber optic scanner mounted with a slide comprising an array of a subject library, comprising:
labeling the array with a bioactivity (affinity or catalysis) marker to generate fluorescent labels on target beads comprising target monomers; and
fluorescent imaging the array with the scanner, preferably wherein the labels provide multiple fluorescent wavelengths, and the imaging comprises optical filtering, reducing background signaling and false positives.

In one embodiment we use a laser (e.g. 488 nm) for excitation of the fluorophores, and then use band pass filters to split the emitted light into two or more channels (e.g. 520 nm and 580 nm), wherein one channel (e.g. 520nm light) represents background light and the other (e.g. 580nm) is our signal light. A calculation of the ratios of these two signals is used to select active polymers.

In another embodiment, we label a non-target molecule (e.g. bovine serum albumin or a protein related to our target) with a different wavelength emitting fluorophore. The ratio of the on-target vs. off-target wavelength signals is then used to select polymers with activity and specificity.

In another aspect the invention describes a method of using a subject library comprising:
fluorescence assaying the library to detect a candidate bead based on bioactivity of the corresponding monomer;
isolating the candidate bead from the assayed library;
cleaving polymers from the isolated candidate bead; and
structurally analyzing the cleaved polymers.

In another aspect, the invention provides a non-natural polymer of a distinct bioactive and sequence pre-defined branching and folding in tertiary structures, wherein the monomer is a dihydroisoquinolinone monomer selected from: 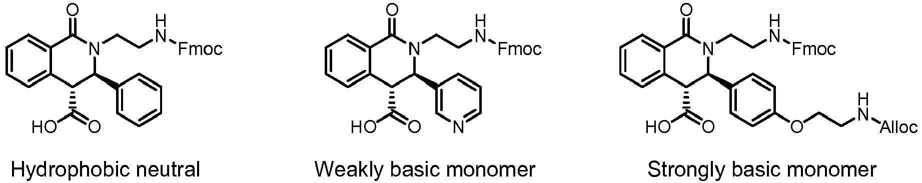 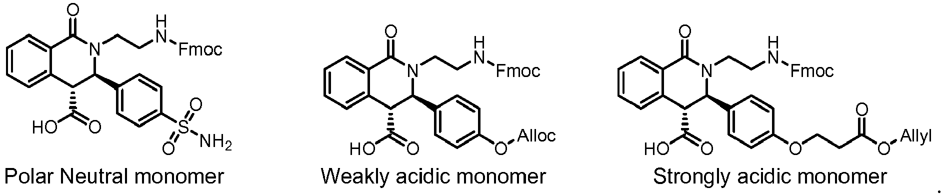 In embodiments:
the polymer is a polyamide, a vinylogous polymer, vinylogous polyamide, or an ester;
the polymer is polymerized in a coupling reaction selected from Wurtz reaction, Glaser coupling, Ullmann reaction, Gomberg-Bachmann reaction, Cadiot-Chodkiewicz coupling, Pinacol coupling reaction, Castro-Stephens coupling, Gilman reagent coupling, Cassar reaction, Kumada coupling, Heck reaction, Sonogashira coupling, Negishi coupling, Stille cross coupling, Suzuki reaction, Hiyama coupling, Buchwald-Hartwig reaction, Fukuyama coupling, and Liebeskind-Srogl coupling;
the polymer or polymerizations is selected from sulphonamides, reductive aminations, peptoid linkages, Suzuki couplings, phospoamidite couplings, and radical cross coupling;
the polymerization combines amide bond coupling methodologies with peptidotriazole branch points introduced using chemically orthogonal copper-catalyzed cycloadditions (click-reactions), and/or
the polymers comprise intrinsically self-readable molecules via intrinsically incorporated isotopically-coded mass-spectroscopy tags or barcodes.

In another aspect the invention provides a library of beads configured for high-throughput drug screening, each of the beads comprising affixed non-natural polymers as disclosed herein.

In another aspect the invention provides a fiber optic scanner mounted with a slide bearing fluorescent beads, preferably a scanner comprising:
an imager stage having a planar surface for supporting a sample comprising the slide;
a bifurcated light path having two fiber optic bundles, each bundle having a first end arranged to define an input aperture for viewing the sample on the imager stage, and a distal bundle end arranged to define an output aperture disposed away from the imager stage;
a scanning source arranged to scan a beam along a path that is perpendicular to the sample on the imager stage and closely adjacent to both bundles of the bifurcated light path such that a substantially circular spot of illumination provided by the scanning source on the imager stage sample provides a light signal at least a portion of which is received by the input aperture of each bundle and transmitted via the bifurcated light path to the output aperture;
a photodetector arranged to detect the light signal at the distal end; and
a processor that processes the light signal detected by the photodetector, wherein each bead comprises affixed non-natural polymers of a distinct bioactive monomer with sequences pre-defined branching and folding in tertiary structure, wherein the monomer is a dihydroisochinolinone.

In another aspect the inventiondescribes a method for imaging a sample comprising the slide bearing fluorescent beads disclosed herein, the method comprising:
supplying a substantially circular beam of radiation perpendicular to the sample;
maintaining the perpendicular direction of the radiation beam as it sweeps along a scan path on the sample;
reflecting at least some light produced by beam interaction with the sample in a direction away from the sample;
collecting light produced by beam interaction with the sample in at least one proximate element of an array of fiber optic first ends;
detecting collected light at a selected output region; and
coordinating sweeping, moving and detecting to generate an array of picture elements representative of at least a portion of the sample.

In another aspect the invention provides a method of using the fiber optic scanner mounted with a slide bearing fluorescent beads disclosed herein, the method comprising:
fluorescent imaging the beads with the scanner, and preferably:
fluorescent imaging the beads with the scanner to detect a candidate bead;
isolating the candidate bead from the beads; and
analyzing a function or structure of the candidate bead.

The invention specifically provides all combinations of the recited embodiments, as if each had been laboriously individually set forth.

### Brief Description of the Figures

Fig. 1. Design strategy for polyamide monomers that mimic protein secondary structure features.
Fig. 2. Scheme for the synthesis of polyamides with sequence-defined branching.
Fig. 3. Non-natural polymer bead configuration.
Fig. 4. Sequencing approach for NNP MS fragmentation
Fig. 5. Library Design for Facile and Rapid Sequencing; Self Readable NNPs
Fig. 6. Isotopically-Coded MS Tags for Sequence Determination

### Detailed Description of Particular Embodiments and Examples Thereof

The invention provides methods of affinity reagent and catalyst discovery employing a library on a bead HTS platform and fiber-optic array scanning technology.

We polymerize bioactive monomers to form non-natural polymers of the bioactive monomers, and attaching these to microbeads (1, 2, 5 or 10 to 10, 20, 50, 100, 200, 500 or 1000 um diameter). In aspects, the invention provides novel non-natural monomers for the synthesis of new polymers, incorporating secondary structural molecular scaffolds and branched structures to maximize the discovery of affinity reagents and catalysts, and a revolutionary high through screening (HTS) platform that can screen a 10⁸ member library in less than 5 minutes.

Synthesis of Monomers and Libraries of Polymers. Utilizing well-established solid-phase peptide chemistry techniques we generate libraries of polyamides with unique, non-natural features such as non-natural amino acid monomers with unique configuration and functionality, non-peptidic scaffolds that mimic features of protein secondary structures, and sequence defined branched structures.

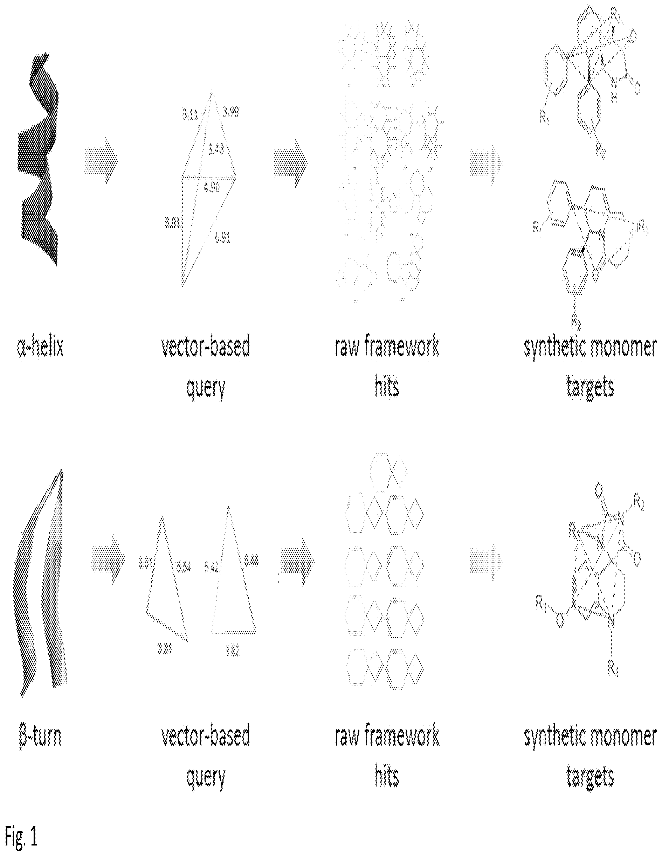

Small Molecule Templates of Secondary Structure Features. Only a small percentage of the amino acids in a protein structure are directly involved in binding interactions or catalytic functions; most are primarily structural determinants that position the interacting residues into proper orientation. The invention provides a strategy for mimicking secondary structure elements using small-molecule templates. In an embodiment, the distances and angles associated with secondary structure elements are modeled as vectors and used to query an enumerated virtual framework library. Hits are then queried across 3D structure databases and manually inspected to identify synthetic monomer targets (Fig. 1). We have designed and validated templates that mimic the angles and side chain projections in α-helixes and β-turns, both of which have been prepared on gram scale [3,4]. By incorporating these templates into polyamide chains, we can mimic the spatial orientation of bioactive structural features with a conformationally restrained scaffold. These monomers also have the capacity for robust combinatorial derivitization, which permits the incorporation of non-natural functional groups into bioactive polymers.

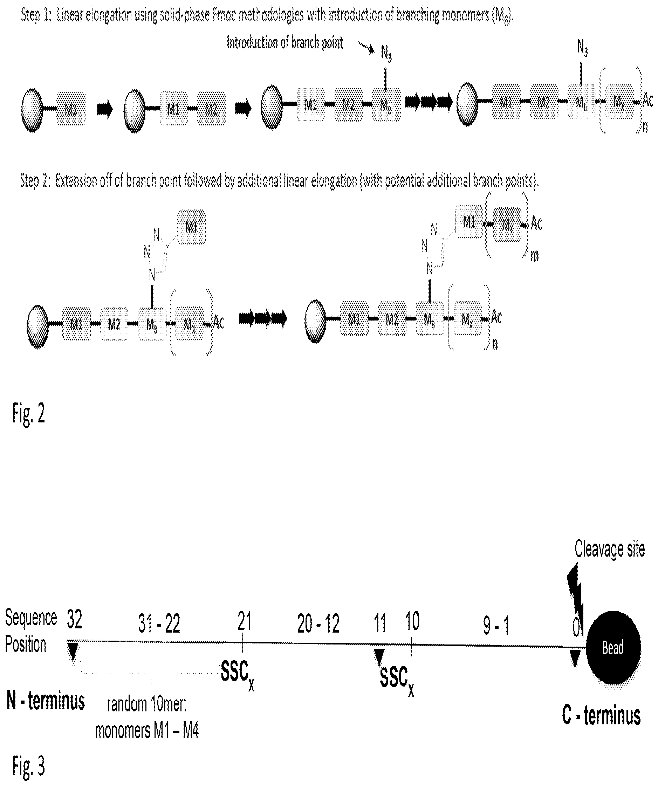

Incorporation of Branching Monomers. Sequence-defined branched polyamides form structured and folded tertiary structures that have unique bioactive properties due to the reduced degrees of spatial freedom. Another benefit of a branched polyamide over a linear polymer is the exponential increase in sequence diversity from a fixed number of monomeric units. Sequence-defined branched polyamides have the chemical properties of proteins but the extraordinary capacity for diversity of polysaccharides. In an embodiment, our approach for accessing branched polyamides uses standard amide bond coupling methodologies combined with peptidotriazole branch points introduced using chemically orthogonal copper-catalyzed cycloadditions ("click-reactions") (Fig. 2).

Design and Synthesis of Non-Natural Amino Acid Monomers. The invention provides non-natural monomers that promote the folding of polyamides into functional conformations. In addition to the above approaches, we have designed non-natural amino acid monomers with backbone dihedral angles outside of those typically found in Ramachandran plot. These amino acids include β- and γ-amino acid variants of natural amino acids that form compact structured conformations. We have designed using molecular dynamics non-natural polypeptides with only four monomers that fold into compact secondary structures assessed by 1D and 2D NMR [5]. All of the monomers are readily synthesized to >98% purity by HPLC and structures assigned unequivocally using 1D/2D NMR, MS and CD. Examples of non-natural monomers that we have made on >1 gram scale to ≥98% purity [1-3] include:

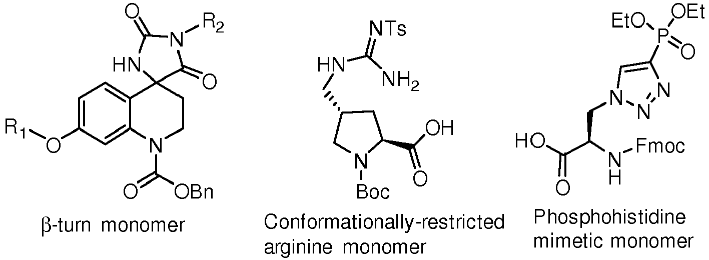

Computation Design and Optimization. The invention provides computational chemistry tools, analogous to those for the modeling of peptide and protein structure and function, to model the non-natural building blocks, polymers and library designs. Development criteria include:
Parameterization of non-natural monomers;
Quantum Mechanics (QM) calculations on building blocks to derive accurate charges, bond lengths, and angles;
QM calculations on dimers and trimers of monomers to derive dihedral preferences.

Validation of computational tools on polymers with these building blocks;
Where possible, extract data set from the Protein Data Bank (PDB) of pseudo-peptides with similar building blocks;
Run relevant modules of tools with the parameters from (1) on the data set and structural data (NMR, X-ray) from initial hit;
Application to the design of novel bioactive polymers;
Design bias libraries with sequences that are more likely to fold and present binding or catalytic functionality in novel and diverse ways.

Production of Large (>108+) Combinatorial Libraries. To accommodate the enormous theoretical sequence space of non-natural polymers, libraries of >108-12 polymers are synthesized using a combinatorial split and pool synthetic strategy [6]. An embodiment utilizes established solid phase synthesis methods, wherein polyethylene glycol (PEG)-grafted microbeads is used as solid support. Monomeric units are added using primarily Fmoc chemistry methods and performed in 96-well filtration manifolds. Reagent addition, washing and vacuum filtration steps are performed using either a PerkinElmer Janus or Biomek Fx liquid handling workstation. Upon addition of the final monomeric unit, the polymers are deprotected on-bead, washed and dried. Since the majority of our coupling reactions are based on established Fmoc chemistry, we use existing protocols for standard peptide couplings and optimize reaction conditions for steps that include non-natural monomeric units.

In embodiments polyamides that range from 20-30 monomeric units are used to determine a minimal length capable of forming well-folded structures. We have shown that though molecular dynamics simulations it is possible to design natural polypeptides with only four monomers that fold into compact secondary structures assessed by 1D and 2D NMR [5]. Libraries may also be produced with both random variations [7] as well as positional scanning approaches [8].

Screening non-natural polymer libraries to identify binders and catalysts; cancer diagnostics. Circulating tumor cells (CTCs) are individual cancer cells that are shed by tumors into the blood stream and are thought to contribute to the process of metastasis. CTCs phenotypes are representative of the status of the primary tumor and as such they provide, through a simple blood draw, a "liquid biopsy" of the entire tumor status of a cancer patient-potentially a very powerful diagnostic tool to non-invasively monitor and direct cancer treatment. The major challenge in identifying CTCs is that they are extremely rare (>1 cell in 5 million); however, an SRI platform called the Fiber-optic Array Scanning Technology (FASTTM) cytometer, which is based on the concept of "Xeroxing" a blood sample with a scanning laser and collecting a high resolution capture image of the sample using a densely packed fiber optic array bundle [9].

In the FAST process blood samples are plated on a glass slide with the footprint of a standard microtitre plate. The slide is coated with a poly-lysine graft that both serves to ensure the formation of a mono-dispersed cell layer and to fix the cells to the glass surface. The cellular monolayer on the glass slide is then treated with fluorescently labeled CTC targeting agents and is scanned with the FAST system. Only 60 seconds are needed to create a digital image of the location of the fluorescently labeled CTCs present on the slide. This is an extraordinarily sensitive system, which we have demonstrated to reliably detect single digit numbers of CTCs on glass slides containing >25,000,000 white blood cells. The system is well validated and robust: SRI has processed thousands of blood samples on the FAST system, which is currently included as a diagnostic platform in human clinical trials validating CTC analysis in cancer care.

Once their location has been identified by the FAST system, we routinely pick individual cells from the glass slide using an aspiration system which then transfers the individual cells in buffer to 384- or 1536-well plates for further processing.

FAST high throughput screening (HTS) of large combinatorial libraries: an extremely rapid and sensitive screening platform which can screen biopolymer libraries of >100,000,000 compounds (>10⁸) in just 4 minutes.

To facilitate on-bead screening, compounds were synthesized on Tentagel-type resins, which contain a polystyrene bead core with a PEG graft co-polymer surface. The PEG coating facilitates display of compounds bound to the bead surface for screening in aqueous buffer. Our approach provides revisiting on-bead solid phase screening with a plurality of innovative approaches; for examples:
Beads can be the same size as cells: Beads for solid phase screening vary from 5-500 microns in diameter. White blood cells screened on the FAST platform typically range from 8-30 microns. While the trend has been to synthesize compounds on larger beads to maximize compound yield, with on-bead screening we only need enough polymer to (i) demonstrate affinity or catalytic activity and (ii) identify the polymer molecular structure once selected as a preliminary hit. By synthesizing libraries on smaller beads (diameter in the range of -10-20 microns), we can synthesize a 100,000,000-member library on as little as 250-500mg of resin.

Beads can be screened using the FAST platform: A 25,000,000-member on-bead library can be screened using a fluorescence-based assay on a glass slide like that used for CTC screening. Once polymer libraries have been synthesized on solid support they are dispersed on FAST glass slides, treated with fluorescently labeled target agent and screened on the FAST scanner for affinity or enzyme activity. The SRI FAST system can scan and identify hits in a 108-member library in 4 mins (60 sec per slide).

Beads can be selected for characterization: Using the same cell picking systems developed for FAST CTC picking, we can pick selected beads from glass slides and deliver them to 384- or 1536-well plates for resin cleavage and characterization by LC/MS/MS (see sequencing section below). Individual cell picks take 15-20 sec per cell. For example, assuming 100 hits are detected on a plate, bead picking will take only 33 mins, or ∼2 hr for the entire 108 library.

We have scaled this example to a library of 10⁸, but the entire process is readily expandable to larger libraries (1¹⁰⁻¹²) or multiple diverse libraries. The process is also readily applied to screening binding to biological targets such organismal coat fragments or proteins, oligosaccharides, proteins, DNA/RNA sequences, cytokines or peptides - essentially any agents that can be tagged with a fluorescent label and directly applied to the library on the slides for read out as described above. For some small molecules, tagging with fluorescent labels can significantly affect the physicochemical properties of the agent and its binding profile, so in an alternative approach we use a fluorescent resonance energy transfer (FRET) assay by incorporating fluorescent and quenching labels at the beginning and end of each library compound synthesized, and then use FAST to identify modulation of fluorescence of individual beads on the slide before and after application of agent. The FRET assay and fluorophores may be optimized for this type of assay, but the FAST scanner has the required sensitivity to detect even minor changes in fluorescence intensity and wavelength.

Sequencing and characterizing non-natural polymers. In an embodiment we use 10-20 micron diameter PEG-grafted polystyrene beads to synthesize large libraries. Typically these types of resins have a loading capacity of 0.2-0.3 mmol of compound per gram of resin. There are approximately 250 million 20-micron beads in a gram of resin, which equates to ∼1 pmol of compound per bead, assuming 100% success for synthesis, cleavage and isolation of product from bead. Even assuming a worst-case scenario of only a 1% yield a 30mer polymer from a single bead with a maximum loading of 1 pmol would produce 10 fmol of product. This amount of product can be readily identified by 1D LC/MS/MS analysis.

A database containing all possible sequences of the library may be constructed and used for identifying biopolymers from MS and MS/MS data. From a single compound on a bead there are only a small number of [MS, MS/MS] spectral pairs to deconvolute from other matching MS library members through fragmentation pattern data.

Validation. Following sequencing, hits may be confirmed by resynthesis and a secondary solution-based binding assay including measure of Kd. Conformational analysis of a series of affinity reagent hits-alone and bound to the target agent-by NMR or X-ray crystallography enables refining predictive models, such as through steps:
Compare bulk (1D) and structural (3D) properties of hits versus non-hits
Analyze conformational and target-binding behavior of hits versus non-hits
Cluster library based on calculated properties
Focus on clusters in which hits are significantly overrepresented
Derive structure-activity model

This ensemble can be used to create models for classes of novel secondary structures represented in binding motifs found in the screening, and in next generation library design and to optimize affinity reagents for binding to specific target agents through structure-activity relationship (SAR) mapping.

### References

1. Kee J-M, Villani B, Carpenter LR, Muir TW (2010) Development of Stable Phosphohistidine Analogues. J Am Chem Soc 132: 14327-14329.
2. Webb TR, Eigenbrot C (1991) Conformationally restricted arginine analogs. J Org Chem 56: 3009-3016.
3. Webb TR, Jiang L, Sviridov S, Venegas RE, Vlaskina AV, et al. (2007) Application of a Novel Design Paradigm to Generate General Nonpeptide Combinatorial Templates Mimicking β-Turns: Synthesis of Ligands for Melanocortin Receptors. J Comb Chem 9: 704-710.
4. Webb TR, Venegas RE, Wang J, Deschenes A (2008) Generation of New Synthetic Scaffolds Using Framework Libraries Selected and Refined via Medicinal Chemist Synthetic Expertise. J Chem Inf Model 48: 882-888.
5. Song B, Kibler P, Malde A, Kodukula K, Galande AK (2010) Design of Short Linear Peptides That Show Hydrogen Bonding Constraints in Water. J Am Chem Soc 132: 4508-4509.
6. Salmon SE, Lam KS, Lebl M, Kandola A, Khattri PS, et al. (1993) Discovery of biologically active peptides in random libraries: solution-phase testing after staged orthogonal release from resin beads. Proc Natl Acad Sci U S A 90: 11708-11712.
7. Menendez A, Scott JK (2005) The nature of target-unrelated peptides recovered in the screening of phage-displayed random peptide libraries with antibodies. Anal Biochem 336: 145-157.
8. Houghten RA (1993) The broad utility of soluble peptide libraries for drug discovery. Gene 137: 7-11.
9. Liu X, Hsieh HB, Campana D, Bruce RH (2012) A new method for high speed, sensitive detection of minimal residual disease. Cytom Part A 81A: 169-175.

### Additional Aspects of the Disclosure

In an aspect, the disclosure incorporates:
Drug-like monomers based on functionally rich molecular frameworks assembled into non-natural polymers (NNPs) with extraordinary function; and/or
Fiber optic array scanning technology (FAST) performs fluorescence-based screening of 10⁸⁻¹⁰ member libraries in <5, <10 or <30 minutes

NNPs designed as intrinsically self-readable molecules using MS "barcodes" incorporated into sequence structures

Structural analyses of novel bioactive NNPs reveal modalities for molecular recognition.

Design of non-natural monomers. Criteria for developing drug-like monomer scaffolds include: stable, chiral, synthetically-scalable, minimal molecular weight, moderate flexibility, functional group density, coupled with reliable chemistry, and orthogonal side chain protecting groups, etc. These criteria are used to produce monomer sets, such as exemplified by candidate sets 1-3, wherein -OH/Cl and Fmoc positions yield monomer chain links:

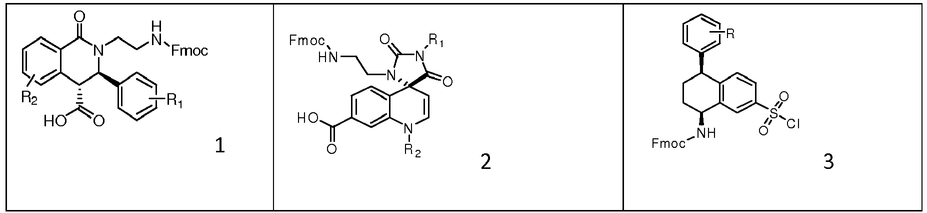

Monomer sets may be designed to sample different backbone torsional angles to potentially explore new types of polymer folding geometries.

Design and synthesis of monomer set 1,
dihydroisoquinolinones:

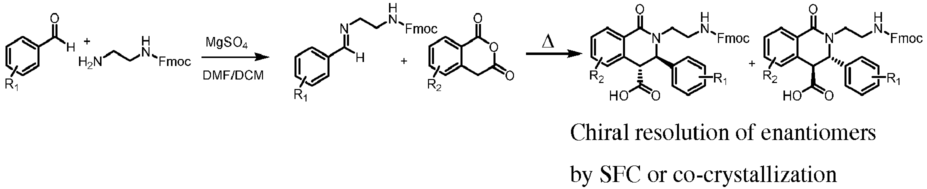

### Exemplary monomers

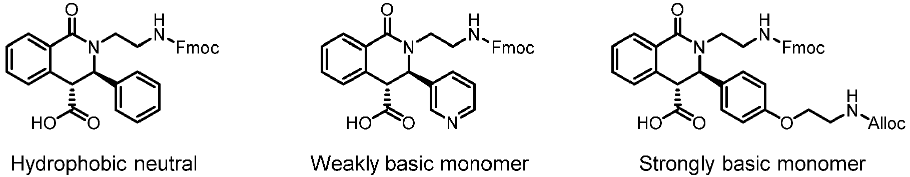

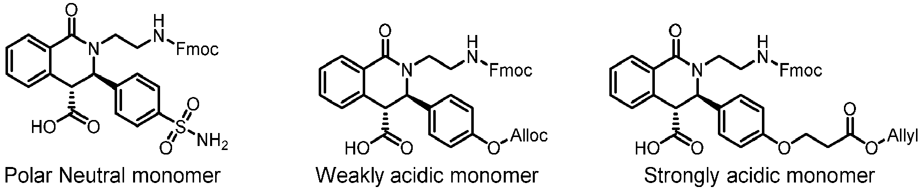

Cleavable secondary structure constraints; inducing tertiary structure

Specialized monomers, secondary structure constraints" (SSCs) are employed to induce tertiary structures into NNPs. SSC's may also be designed to be cleavable to facilitate MS-based sequencing of the NNPs:

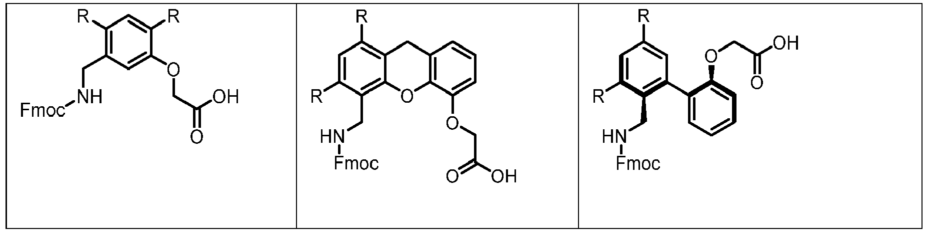

| Gamma turn 120 degrees | Beta-turn 180 degrees | Helix 270 degree turn |
|---|---|---|
| 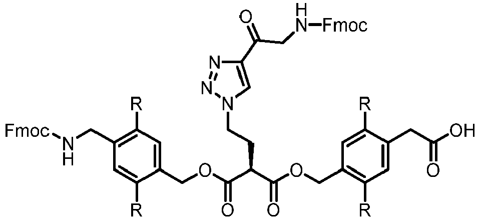 | | |
| Branching monomer | | |

NNP sublibrary configurations. Configurations of ∼30mers with monomers and SSCs are constructed to maximize diversity; Fig. 3. Null spacer monomers (e.g. beta Ala) may be incorporated to simplify sequence and synthesis.

| Num. monomer /position | Num. positions | SSC #1:Num. SSCs | SSC #2:Num. SSCs | Sub library size | Configuration Description |
|---|---|---|---|---|---|
| 4 | 12 | 3 | 3 | 1.51x10⁸ | 4 monomers per position in 12 positions; 3 SSCs in 2 positions |
| 2 | 24 | 3 | 3 | 1.51x10⁸ | 2 monomer per position randomly selected from 4 monomers, in 24 positions; 3 SSCs in 2 positions |
| 2 | 25 | 3 | 3 | 1.01x10⁸ | 2 monomer per position randomly selected from 4 monomers, in 25 positions; 3 SSCs in position #1, branched SSC in #2. |

| | | | | | |
|---|---|---|---|---|---|
| Total Library = 4.03x10⁸ | | | | | |

FAST screening of libraries on beads using a well-fee plate, wherein compounds are attached to beads, rather than wells, and the number of assays/plate is determined by bead size (e.g. diameter); for examples, 1x10⁵⁻⁶ for 50-100um beads; 25x10⁶ for 10um beads.

FAST mega-screening enables screening of millions of compounds per minute. In an exemplary assay: combine fluorescent target with 10⁸ bead library in tube, incubate and wash, transfer ∼25M 10um beads/slide, FAST scan (~1 min/plate), rank & determine signal intensity, wherein dual wavelength can reduce false positives, automated bead picking and transfer to 384-well plate, cleave NNPs from beads and sequence.

Screening: Create and demonstrate a generalizable screening strategy and platform.

Bead dispersion and attachment to slides - fluorescent assay, binding or enzymatic activity - FAST locate hits - ADM verify hits - bead picking - sequence.

Characterization of Hits. Hits are characterized for binding affinity or catalytic activity
Affinity reagents: Binding affinities are determined by surface plasmon resonance (SPR) to measure association and dissociation constants (ka,kd)
Catalysts: Michaelis Menten model is used to determine Vmax (maximal velocity), Km (Michaelis constant, ½ Vmax). The catalytic constant (kcat)
Differential scanning fluorimetry (DSF), Dynamic Light Scawering (DLS), analytical Size Exclusion Chromatography (anSEC)
folding, complex formation, solubility, stability
x-ray structure determination for up to three hits per target

### MS Identification of NNPs

| bead diameter | theor. moles NNP/1 bead* | MS + MS/MS detection sensitivity |
|---|---|---|
| 90 micron | 21 pmol | operable |
| 10 micron | 63 fmol | operable (fast duty cycle proteomics) |

| | | |
|---|---|---|
| *with 10% NNP isolation yield. | | |

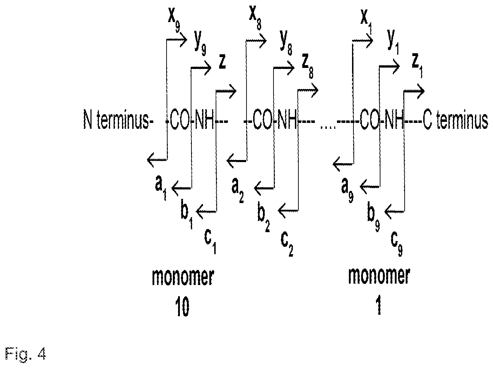

Sequencing approach for NNP MS fragmentation: detect sequencing ions: a, b, c, x, y, z; additional backbone fragmentations for complex NNP backbones; Fig. 4.

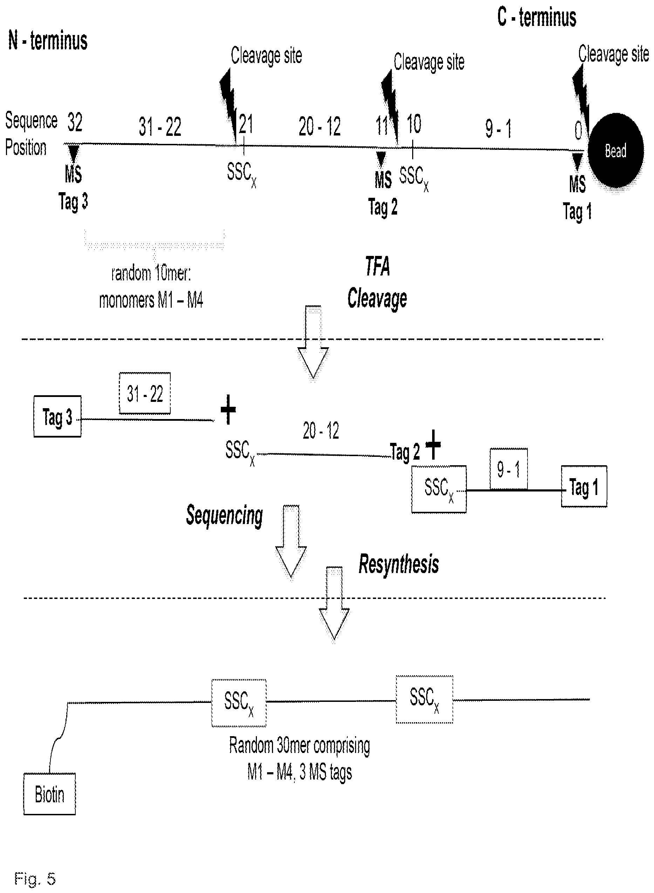

Library design for facile and rapid sequencing; self-readable NNPS is shown in Fig. 5.

### Isotopically-coded MS tags for sequence determination:

| | |
|---|---|
| 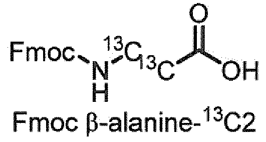 | 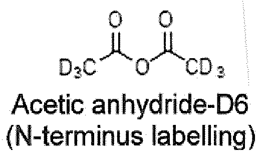 |
| [M + 2] MS Tag | [M + 3] MS Tag |
| 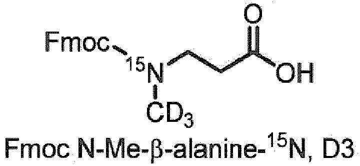 | 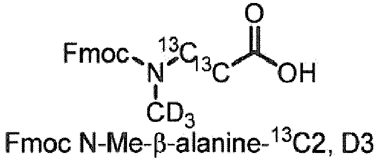 |
| [M +4] MS Tag | [M + 5] MS Tag |

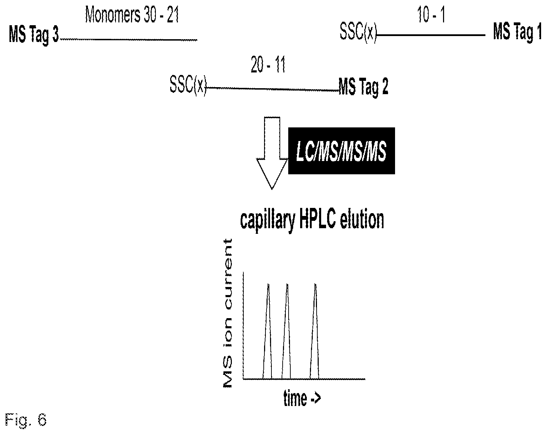

Incorporating MS tags enhances sensitivity of sequencing and allows for reconstruction of full sequence from smaller polymer fragments, Fig. 6.

### Multiple and/or alternative fragmentation modes may be used:

| fragmentation method | mechanism | main ions (peptides) |
|---|---|---|
| collision-induced dissociation (CID) | RF-enhanced collisions, vibrational fragmentation | b, y, few a |
| high energy CID (HCD) | collision cell higher energy vibrational fragmentation | different/more b, y, low mass ions |
| electron transfer dissociation (ETD) | free radical cleavage | c, z; better for higher charge states |

Sequencing may be optimized, e.g. optimize each fragmentation method for each class of NNPs; create more sequencing ions: use multiple fragmentation methods; MSn; library.

Design; compare identified sequencing ions for known NNPs using each method, combinations of methods; support vector machine learning analysis, etc.

### Automated Sequencing of Self Readable Polymers

| engine | strengths | challenges |
|---|---|---|
| de novo sequencing | no database, direct sequence reconstruction from MS/MS data | dense sequence space; often poor success rate, trouble distinguishing close sequence variants |
| custom | analyze multiple fragmentation modes; combine more than one engine | dense sequence space; implement for NNPs short peptides |
| database search | most successful analyzing peptide fragmentation data | dense NNP sequence space; searching databases when > 1e10 sequences |

Implementation criteria: software analysis of complex data, adapt, compare different search engines using NNP fragmentation data; analyze initial data sets from known NNPs with best-performing proteomics engines in above categories; use combinations of engines; combined fragmentation data, MSn, etc; ; library design e.g. no isobaric monomers.

It is understood that the examples and embodiments described herein are for illustrative purposes only and that various modifications or changes in light thereof will be suggested to persons skilled in the art within the scope of the appended claims.

## Claims

1. A library of beads configured for high-throughput drug screening, each bead comprising affixed non-natural polymers of a distinct bioactive monomer with sequences pre-defined branching and folding in tertiary structure, wherein the monomer is a dihydroisoquinolinone.

2. The library of claim 1 further comprising incorporated in each of the polymers secondary structure constraint (SSC) monomers which impose turns and thereby induce the tertiary structure, wherein the SSC monomers are acid cleavable to facilitate mass-spectroscopy based sequencing of the polymers.

3. The library of claim 1 configured in an array on a slide.

4. The library of claim 1, wherein the dihydroisoquinolinone monomer is selected from:

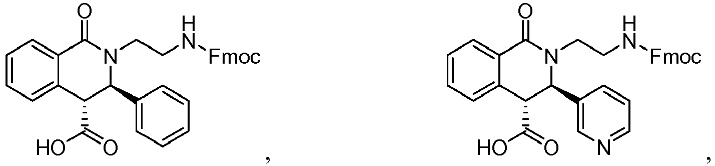

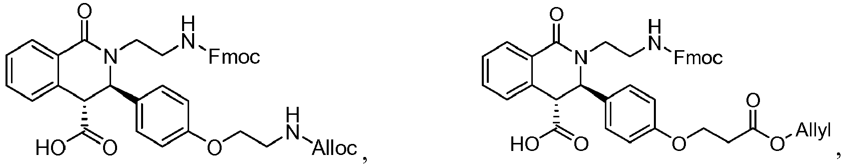

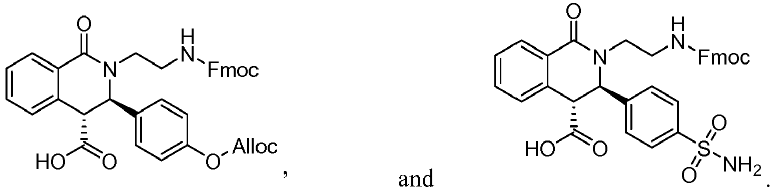

5. A non-natural polymer of a distinct bioactive monomer with sequences pre-defined branching and folding in tertiary structure, wherein the monomer is a dihydroisoquinolinone monomer selected from:

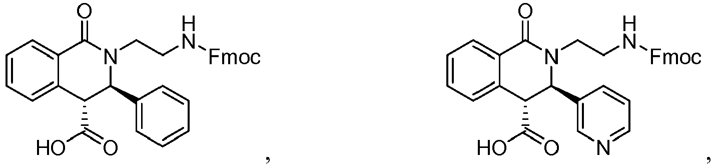

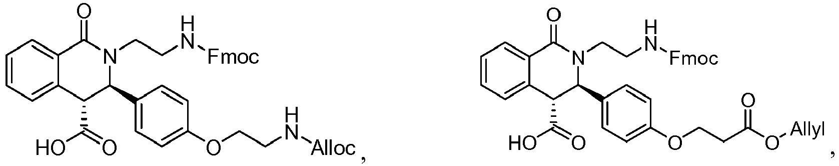

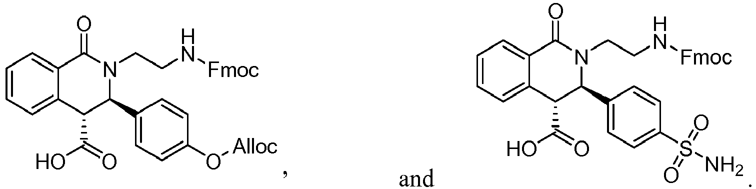

6. The polymer of claim 5, further comprising incorporated in each of the polymers secondary structure constraint (SSC) monomers which impose turns and thereby induce the tertiary structure, preferably wherein the SSC monomers are acid cleavable to facilitate mass-spectroscopy based sequencing of the polymers.

7. A fiber optic scanner mounted with a slide bearing fluorescent beads, the scanner comprising:
(a) an imager stage having a planar surface for supporting a sample comprising the slide;
(b) a bifurcated light path having two fiber optic bundles, each bundle having a first end arranged to define an input aperture for viewing the sample on the imager stage, and a distal bundle end arranged to define an output aperture disposed away from the imager stage;
(c) a scanning source arranged to scan a beam along a path that is perpendicular to the sample on the imager stage and closely adjacent to both bundles of the bifurcated light path such that a substantially circular spot of illumination provided by the scanning source on the imager stage sample provides a light signal at least a portion of which is received by the input aperture of each bundle and transmitted via the bifurcated light path to the output aperture;
(d) a photodetector arranged to detect the light signal at the distal end; and
(e) a processor that processes the light signal detected by the photodetector,
wherein each bead comprises affixed non-natural polymers of a distinct bioactive monomer with sequences pre-defined branching and folding in tertiary structure, wherein the monomer is a dihydroisoquinolinone.

8. The fiber optic scanner of claim 7, wherein the dihydroisoquinolinone monomer is selected from:

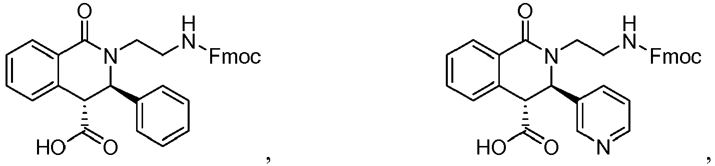

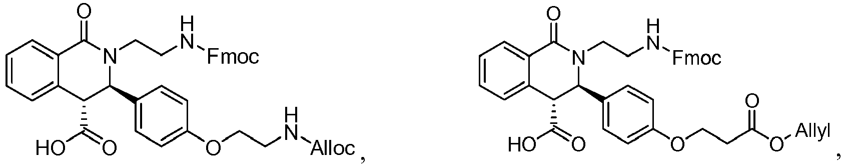

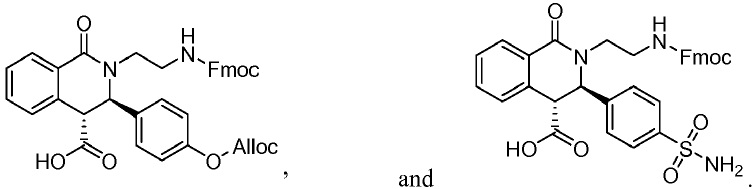

9. A method for imaging a sample comprising a slide bearing fluorescent beads, the method comprising:
(a) supplying a substantially circular beam of radiation perpendicular to the sample;
(b) maintaining the perpendicular direction of the radiation beam as it sweeps along a scan path on the sample;
(c) reflecting at least some light produced by beam interaction with the sample in a direction away from the sample;
(d) collecting light produced by beam interaction with the sample in at least one proximate element of an array of fiber optic first ends;
(e) detecting collected light at a selected output region; and
(f) coordinating sweeping, moving and detecting to generate an array of picture elements representative of at least a portion of the sample
wherein each bead comprises affixed non-natural polymers of a distinct bioactive monomer with sequences pre-defined branching and folding in tertiary structure, wherein the monomer is a dihydroisoquinolinone.

10. The method of claim 9, further comprising the steps of:
(a) detecting a candidate bead;
(b) isolating the candidate bead; and
(c) analyzing a function or structure of the candidate bead.

11. The method of claim 9, wherein the dihydroisoquinolinone monomer is selected from:

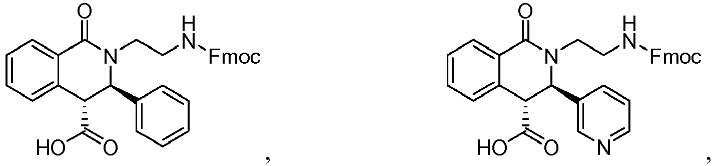

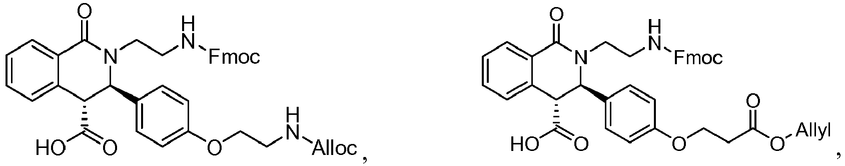

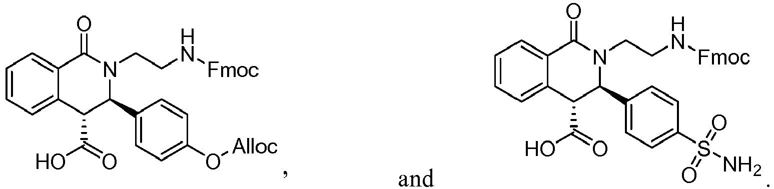

12. The method of claim 9, wherein the polymers further comprise incorporated in each of the polymers secondary structure constraint (SSC) monomers which impose turns and thereby induce the tertiary structure, wherein the SSC monomers are acid cleavable to facilitate mass-spectroscopy based sequencing of the polymers.

## Patentansprüche

1. Bibliothek von Beads, die an ein Wirkstoffscreening mit hohem Durchsatz angepasst ist, wobei jedes Bead befestigte nicht-natürliche Polymere eines eigenständigen bioaktiven Monomers umfasst, die Sequenzen mit in ihrer Tertiärstruktur vorgegebenen Verzweigungen und Faltungen aufweisen, wobei das Monomer ein Dihydroisoquinolinon ist.

2. Bibliothek nach Anspruch 1, ferner auf die Sekundärstruktur beschränkte (SSC) Monomere umfassend, die in jedem der Polymere aufgenommen sind und Windungen einführen und dabei die Tertiärstruktur induzieren, wobei die SSC Monomere durch Säure spaltbar sind, um eine auf Massenspektrometrie basierende Sequenzierung der Polymere zu erleichtern.

3. Bibliothek nach Anspruch 1, konfiguriert als Array auf einer Platte.

4. Bibliothek nach Anspruch 1, wobei das Dihydroisoquinolinonmonomer ausgewählt ist unter:

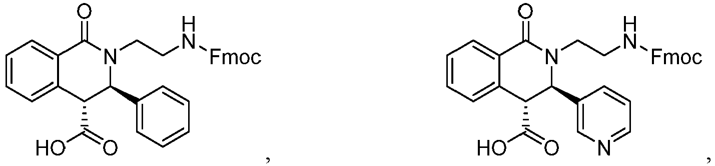

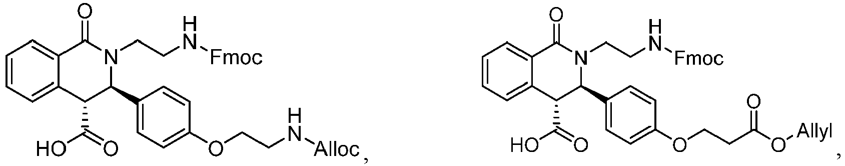

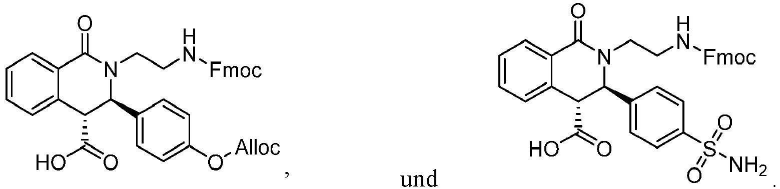

5. Nicht-natürliches Polymer eines eigenständigen bioaktiven Monomers mit Sequenzen, die in ihrer Tertiärstruktur vorgegebene Verzweigungen und Faltungen aufweisen, wobei das Monomer ein Dihydroisoquinolinonmonomer ist, ausgewählt unter:

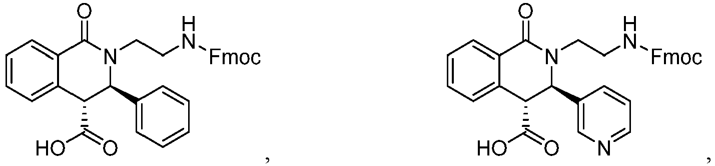

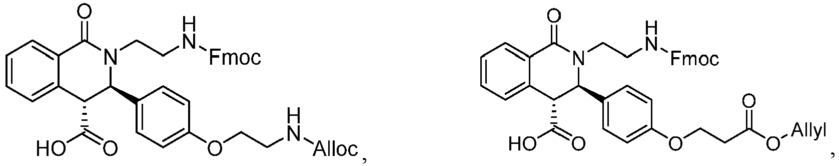

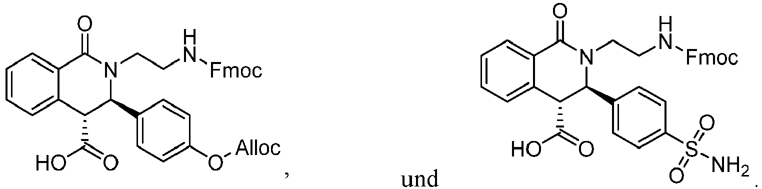

6. Polymer nach Anspruch 5, ferner auf die Sekundärstruktur beschränkte (SSC) Monomere umfassend, die in jedem der Polymere aufgenommen sind und Windungen einführen und dabei die Tertiärstruktur induzieren, vorzugsweise wobei die SSC Monomere durch Säure spaltbar sind, um eine auf Massenspektrometrie basierende Sequenzierung der Polymere zu erleichtern.

7. Faseroptischer Scanner, angeordnet mit einer Platte, die fluoreszierende Beads trägt, wobei der Scanner umfasst:
(a) Eine Imagerstufe mit einer ebenen Oberfläche zum Tragen einer Probe umfassend die Platte;
(b) ein Gabellichtpfad mit zwei optischen Faserbündeln, wobei jedes Bündel ein erstes Ende aufweist, das angeordnet ist, eine Einlassblende zum Betrachten der Probe auf der Imagerstufe zu bestimmen sowie ein distales Bündelende, das angeordnet ist, um eine Auslassblende zu definieren, die von der Imagerstufe entfernt angeordnet ist;
(c) eine Scannerquelle, die angeordnet ist, um einen Strahl entlang eines Pfads zu scannen, der rechtwinkelig zu der Probe auf der Imagerstufe und dicht an beiden Bündeln des Gabellichtpfads verläuft, so dass ein im Wesentlichen runder Leuchtpunkt, der durch die Scannerquelle auf der Probe der Imagerstufe bereitgestellt wird, ein Lichtsignal bereitstellt, von dem wenigstens ein Teil von der Einlassblende jedes Bündels erhalten und über den Gabellichtpfad zu der Auslassblende übertragen wird;
(d) ein Photodetektor, der angeordnet ist, um das Lichtsignal an dem distalen Ende zu erfassen; und
(e) ein Prozessor, der das durch den Photodetektor erfasste Lichtsignal verarbeitet,
wobei jedes Bead befestigte nicht-natürliche Polymere eines eigenständigen bioaktiven Monomers umfasst, die Sequenzen mit in ihrer Tertiärstruktur vorgegebenen Verzweigungen und Faltungen aufweisen, wobei das Monomer ein Dihydroisoquinolinon ist.

8. Faseroptischer Scanner nach Anspruch 7, wobei das Dihydroisoquinolinonmonomer ausgewählt ist unter:

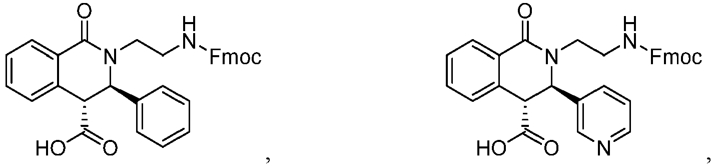

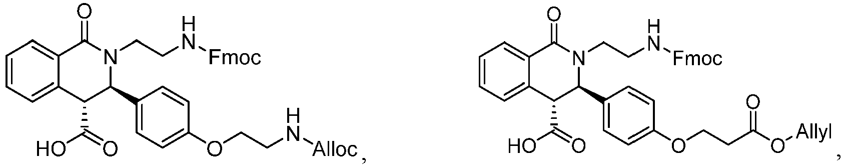

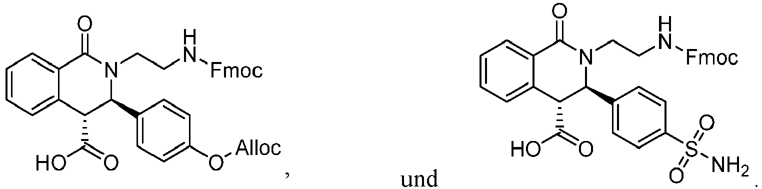

9. Verfahren zum Abbilden einer Probe umfassend eine Platte, die fluoreszierende Beads trägt, wobei das Verfahren umfasst:
(a) Bereitstellen eines im Wesentlichen zirkulären Strahls von Strahlung rechtwinkelig zu der Probe;
(b) Aufrechterhalten der rechtwinkeligen Richtung des Strahlungsstrahls, während er entlang eines Scanpfads die Probe abtastet;
(c) Reflektieren von wenigstens etwas Licht, das durch die Interaktion von dem Strahl und der Probe erzeugt wird, in einer Richtung weg von der Probe;
(d) Sammeln des durch die Interaktion von dem Strahl und der Probe erzeugten Lichts in wenigstens einem proximalen Element eines Arrays aus ersten Enden von optischen Fasern;
(e) Erfassen des gesammelten Lichts in einem ausgewählten Ausgabebereich; und
(f) Koordinieren des Abtastens, Bewegens und des Erfassens, um einen Array an Bildelementen zu erzeugen, der wenigstens einen Teil der Probe darstellt,
wobei jedes Bead befestigte nicht-natürliche Polymere eines eigenständigen bioaktiven Monomers umfasst, die Sequenzen mit in ihrer Tertiärstruktur vorgegebenen Verzweigungen und Faltungen aufweisen, wobei das Monomer ein Dihydroisoquinolinon ist

10. Verfahren nach Anspruch 9, ferner umfassend die Schritte:
(a) Erfassen eines Kandidatenbeads;
(b) Isolieren des Kandidatenbeads; und
(c) Analysieren einer Funktion oder einer Struktur des Kandidatenbeads.

11. Verfahren nach Anspruch 9, wobei das Dihydroisoquinolinonmonomer ausgewählt ist unter:

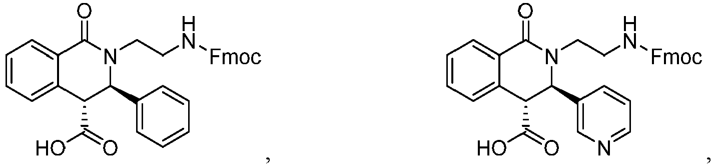

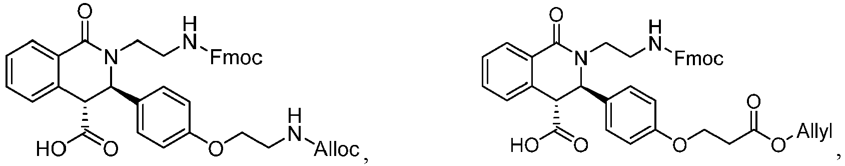

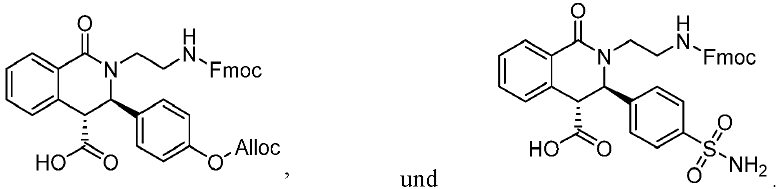

12. Verfahren nach Anspruch 9, wobei die Polymere ferner auf die Sekundärstruktur beschränkte (SSC) Monomere umfassen, die in jedem der Polymere aufgenommen sind und Windungen einführen und dabei die Tertiärstruktur induzieren, wobei die SSC Monomere durch Säure spaltbar sind, um eine auf Massenspektrometrie basierende Sequenzierung der Polymere zu erleichtern.

## Revendications

1. Banque de billes configurée pour le criblage de médicaments à haut débit, chaque bille comprenant appliqués des polymères non naturels d'un monomère bioactif distinct avec des séquences prédéfinies se ramifiant et se repliant en structure tertiaire, dans laquelle le monomère est une dihydroisoquinolinone.

2. Banque selon la revendication 1, comprenant en outre incorporés dans chacun des polymères des monomères à contrainte de structure secondaire (CSS) qui imposent des tours et ainsi induisent la structure tertiaire, les monomères SSC étant clivables par un acide pour faciliter le séquençage des polymères basé sur la spectroscopie de masse.

3. Banque selon la revendication 1, configurée en un ensemble sur une lame.

4. Banque selon la revendication 1, dans laquelle le monomère dihydroisoquinolinone est choisi parmi :

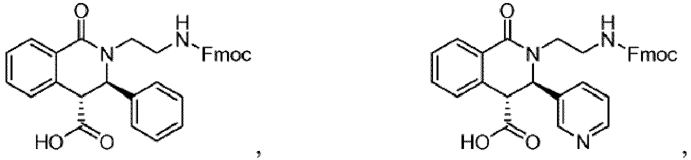

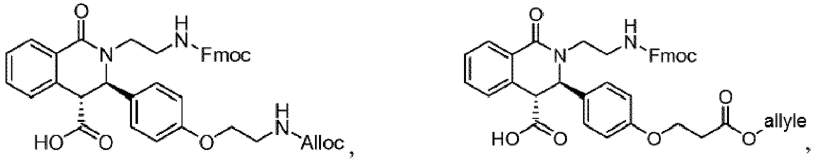

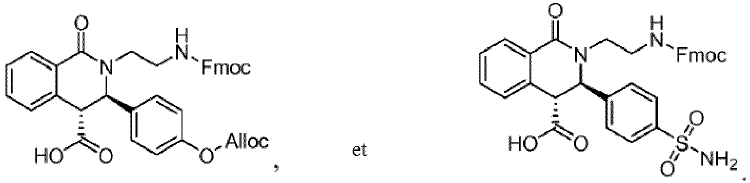

5. Polymère non naturel d'un monomère bioactif distinct avec des séquences prédéfinies se ramifiant et se repliant en structure tertiaire, dans lequel le monomère est un monomère dihydroisoquinolinone choisi parmi :

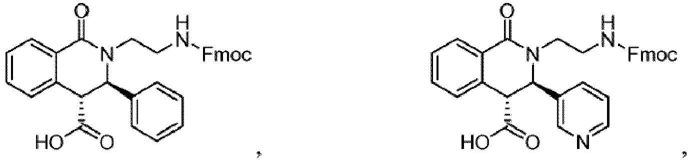

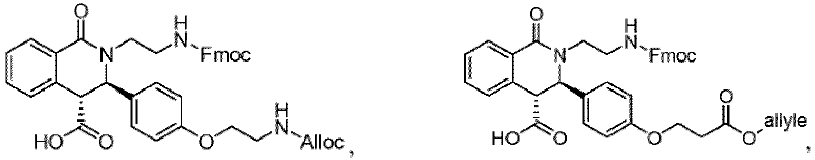

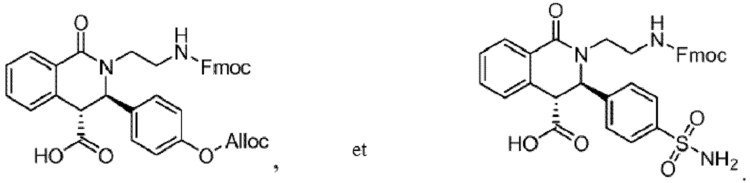

6. Polymère selon la revendication 5, comprenant en outre incorporés dans chacun des polymères des monomères à contrainte de structure secondaire (CSS) qui imposent des tours et ainsi induisent la structure tertiaire, de préférence dans lequel les monomères SSC sont clivables par un acide pour faciliter le séquençage des polymères basé sur la spectroscopie de masse.

7. Dispositif à balayage à fibres optiques monté avec une lame portant des billes fluorescentes, le dispositif à balayage comprenant ;
(a) un étage imageur ayant une surface plane destinée à supporter un échantillon comprenant la lame ;
(b) un chemin optique bifurqué comportant deux faisceaux de fibres optiques, chaque faisceau présentant une première extrémité disposée pour définir une ouverture d'entrée pour la vision de l'échantillon sur l'étage imageur, et une extrémité distale du faisceau disposée pour définir une ouverture de sortie placée à distance de l'étage imageur ;
(c) une source de balayage disposée pour balayer un faisceau le long d'un chemin qui est perpendiculaire à l'échantillon sur l'étage imageur et étroitement adjacent aux deux faisceaux du chemin optique bifurqué, de sorte qu'un point essentiellement circulaire d'illumination fourni par la source de balayage sur l'échantillon de l'étage imageur fournit un signal lumineux dont au moins une partie est reçue par l'ouverture d'entrée de chaque faisceau et transmise via le chemin optique bifurqué à l'ouverture de sortie ;
(d) un photodétecteur disposé pour détecter le signal lumineux à l'extrémité distale ; et
(e) un processeur qui traite le signal lumineux détecté par le photodétecteur ;
dans lequel chaque bille comprend appliqués des polymères non naturels d'un monomère bioactif distinct avec des séquences prédéfinies se ramifiant et se repliant en structure tertiaire, le monomère étant une dihydroisoquinolinone.

8. Dispositif à balayage à fibres optiques selon la revendication 7, dans lequel le monomère dihydroisoquinolinone est choisi parmi :

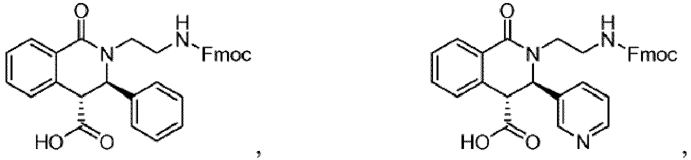

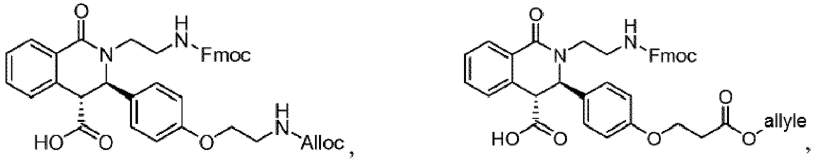

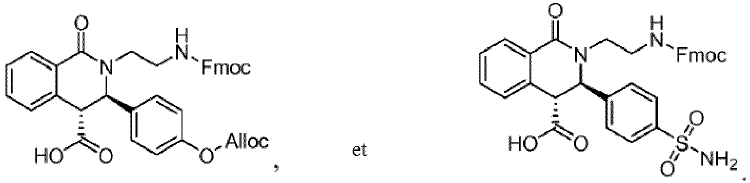

9. Procédé pour l'imagerie d'un échantillon comprenant une lame portant des billes fluorescentes, le procédé comprenant les étapes consistant à :
(a) fournir un faisceau essentiellement circulaire de rayonnement perpendiculaire à l'échantillon ;
(b) maintenir la direction perpendiculaire du faisceau de rayonnement alors qu'il balaie le long d'un cheminement de balayage sur l'échantillon ;
(c) réfléchir au moins une certaine lumière produite par l'interaction du faisceau avec l'échantillon dans une direction s'éloignant de l'échantillon ;
(d) capter la lumière produite par l'interaction du faisceau avec l'échantillon dans au moins un élément, se trouvant à proximité, d'un ensemble de premières extrémités de fibres optiques ;
(e) détecter la lumière captée au niveau d'une région de sortie choisie ; et
(f) coordonner balayage, déplacement et détection pour engendrer un ensemble d'éléments d'images représentatifs d'au moins une partie de l'échantillon
dans lequel chaque bille comprend appliqués des polymères non naturels d'un monomère bioactif distinct avec des séquences prédéfinies se ramifiant et se repliant en structure tertiaire, le monomère étant une dihydroisoquinolinone.

10. Procédé selon la revendication 9, comprenant en outre les étapes consistant à :
(a) détecter une bille candidate ;
(b) isoler la bille candidate ; et
(c) analyser une fonction ou structure de la bille candidate.

11. Procédé selon la revendication 9, dans lequel le monomère dihydroisoquinolinone est choisi parmi :

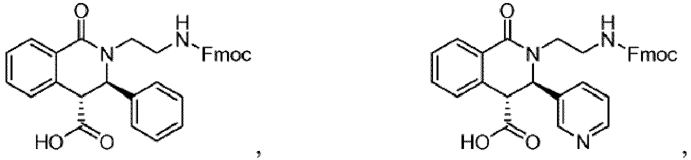

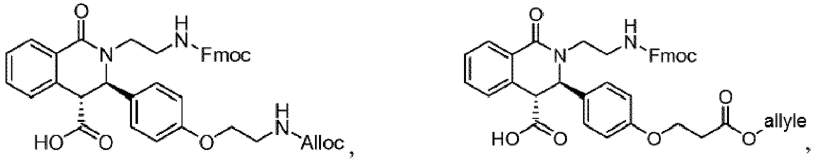

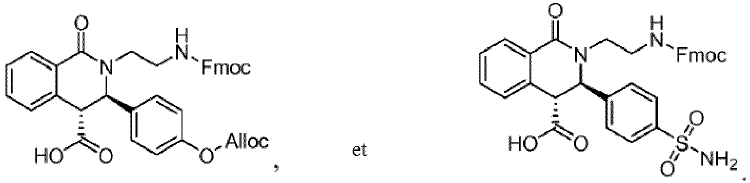

12. Procédé selon la revendication 9, dans lequel les polymères comprennent en outre incorporés dans chacun des polymères des monomères à contrainte de structure secondaire (CSS) qui imposent des tours et ainsi induisent la structure tertiaire, les monomères SSC étant clivables par un acide pour faciliter le séquençage des polymères basé sur la spectroscopie de masse.
